# EUROPEAN PATENT APPLICATION

(11) **EP 0 638 808 A1**
(43) Date of publication of application: **15.02.1995**
(21) Application number: 94112071.9
(22) Date of filing: 02.08.1994
(51) Int. Cl.: G01N 33/84

(54) **Composite reagent for a veterinary diagnostic test**

(30) Priority: 02.08.1993 JP 191201/93
(71) Applicant: Maeda, Hiroshi, Kobe-shi, Hyogo (JP); Okawa, Shozo, Ibaraki-shi, Osaka (JP)
(72) Inventor: Tsutsumi, Kazuhiro, Sakata-gun, Shiga (JP)
(74) Representative: Hansen, Bernd, Dr. Dipl.-Chem.

(57) **Abstract**

A composite reagent, for a veterinary diagnostic test, which contains a color reagent substance to produce a color by reacting with either one or both of magnesium (Mg) ions and calcium (Ca) ions, a polyoxyethylene alkylphenyl ether, and a masking agent for interfering metal ions in a urine of an animal other than the Mg ions and the Ca ions.

## Description

### Background of the Invention

The present invention relates to a composite reagent for a veterinary diagnostic test, which helps in diagnosing or confirming the presence of a urinary calculus and diseases of the urinary tract in animals involving pets such as dogs and cats.

The diseases of the animal urinary tract have prevailed in recent years among many of pets such as dogs and cats, and the incidence of such diseases is now increasing. Major causes of such diseases have been hitherto asserted to lay in that the pets have physical discomfort due to lack of exercise because many of pets are kept indoors by a recent change of urbanized human living environment, and in that the ready-made foods sold for pets are limited in kinds and so biased in nutrition. However, the true causes have not yet been finally determined.

Accordingly, in the field of veterinary medicine, it is required to counteract the expansion of the diseases of the animal urinary tract with a simple method of diagnosing such diseases by external observation. However, in the status quo, an externally observable diagnostic test in itself is not established which meets such a simple method, because there have been found no practical reagent and means which are useful for such a test and can detect the diseases of the animal urinary tract. Incidentally, there was previously an assertion that the diagnosis of the above-mention diseases could be made by measuring the pH value of the urine taken from an animal, on the assumption that an alkaline urine was relevant to such diseases. However, it has been proven that the relationship does not always exist between the alkaline urine and such diseases because phosphorus (P) ions or the like from the pet food affects strongly the urine.

### Summary of the Invention

It is, therefore, an objective of the present invention to provide a composite reagent which can provide a veterinary observable diagnostic test for practical use as a countermeasure against the expansion of the diseases of the animal urinary tract, i.e. to provide a composite reagent which can detect the relevant factors of the diseases of the animal urinary tract from the animal urine quantitatively by a process of chemical color reaction therewith so as to make it possible to diagnose the presence and degree of such diseases correctly by external observation of coloration of a test solution and then try an appropriate measure against such diseases.

It is another objective of the present invention to provide a composite reagent, for a veterinary observable diagnostic test, which can be produced readily in a reduced cost with superiority especially in stability of preservation, reactivity after a long period of preservation, reliability of diagnosis, and handling.

It is a further objective of the present invention to provide a composite reagent, for a veterinary observable diagnostic test, which enables the relevant factors to be exactly determined quantitatively for correct diagnosis also by the use of an apparatus.

With the above and other objectives in view, the present invention provides a composite reagent, for a veterinary diagnostic test, which contains a color reagent substance to produce a color by reaction with either one or both of magnesium (Mg) ions and calcium (Ca) ions, a polyoxyethylene alkylphenyl ether, and a masking agent for interfering metal ions in a urine of an animal other than the Mg ions and the Ca ions.

For a use of the present invention, the veterinary diagnostic test is conducted by treating a small amount of the urine of an animal with a test solution of the composite reagent of the invention. If there are magnesium (Mg) ions or/and calcium (Ca) ions in the animal urine, the composite reagent reacts with the specified metal ions so as to reveal a color of color saturation proportional to the concentration of the specified metal ions. Until now, it has been known clearly that magnesium ions and calcium ions come into existence in the urine of the animal who suffers from urine discharge disorders such as dysuria and urinary retention due to the urinary calculus and diseases of the urinary tract such as hematuria and cystitis. Therefore, by observing the coloration of the test solution, it is possible to diagnose easily the presence of such disorders and diseases and their degree from the color saturation, so that appropriate instructions can be given as to a remedy step, for example, to promote the discharge of the urine by administering diuretics against the slight disorder, or to take a surgical measure against the serious disease. If need be, the Mg ions and the Ca ions are allowed to be exactly determined quantitatively by applying an absorptiometer method to the coloration of the test solution, so that a more correct diagnosis can be made.

On the other hand, it is conceivable to measure metal ions in the urine by an enzymolysis. However, this method is not suitable for practical use on the following grounds and so cannot be adopted actually. More particularly, this method requires a relatively expensive reagent which is prepared through complicated processes, very inferior in stability of preservation, i.e. relatively short in a period of preservation (its effective period being about one month even when preserved in a refrigerator), and relatively low in reactivity and reliability (when used after preservation). Further, the reagent is inconvenient in handling, and a test solution of the reagent is clear so that a quantitative determination is limited only to the use of an absorptiometer method and a simple diagnosis by external observation is impossible.

For a more complete understanding of the above and other features and advantages of the invention, reference should be made to the following detailed description of the invention and the example.

### Detailed Description of the Invention

A composite reagent of the present invention, which is utilized for a veterinary diagnosis test, is formed by mixing a color reagent substance to react with specified metal ions, a polyoxyethylene alkylphenyl ether, and a masking agent for main interfering metal ions other than the specified metal ions in a urine of an animal.

The color reagent substance, which is one constituent of the composite reagent of the present invention, is one which is provided for revealing a color by reaction with magnesium (Mg) ions or/and calcium (Ca) ions. For this color reagent substance, a xylylazo violet-1 (also called, xylidyl blue-1) is preferable from two aspects of stability and reactivity. The xylylazo violet-1 is represented by 1-azo-2-hydroxy-3-(2,4-dimethylcarboxy anilide)-naphtolen-1'-(2-hydroxybenzene-5-sulfonic acid) sodium, and produces a color by chelation in which -OH group in the following structural formula is replaced by magnesium (II) Mg²⁺ , or calcium (II) Ca²⁺ . The produced color is of reddish violet in the alkali range of pH value 11 and more. To the advantage, the use of the xylylazo violet-1 as a color reagent substance enhances greatly the stability and reactivity of the color reagent and the reliability of diagnosis. 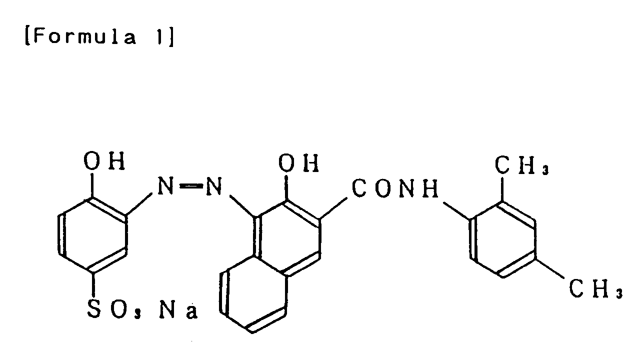
The polyoxyethylene alkylphenyl ether, which is one constituent of the aforementioned composite reagent, is a so-called nonionic surfactant having an amhipathic property, and is one which acts as a solvent for the molecules of fats and proteins in the animal urine so as to enhance the stable coloration of the aforementioned color reagent. In the case where the xylylazo violet-1 is used, as described above, for a color reagent substance, the polyoxyethylene alkylphenyl ether acts as a promotor which helps the composite reagent to dissolve promptly in water. It is desirable that the polyoxyethylene alkylphenyl ether has an isooctyl group as alkyl groups and the number of 9 or 10 in the general formula (CH₂CH₂O)ₙ H of polyoxyethylene.

The masking agent, which is one constituent of the aforementioned composite reagent, is one which is provided for masking main interfering metal ions other than Mg ions and Ca ions in the animal urine, i.e. for preventing the aforementioned color reagent from reacting with the interfering ions so as to allow the coloration action of the color reagent for correct diagnosis. For the masking agent of this type, there are many suitable compounds. Above all, the representative ones are a triethanolamine (TEA) for iron (Fe) (II) ions and irons (Fe) (III) ions; an ethylene glycolbis (2-aminoethyl ether) N·N'-tetraacetic acid (EGTA) for manganese (Mn) ions and nickel (Ni) ions; and a tetraethylene pentaamine (TEPA) for cobalt (Co) ions, nickel (Ni) ions, copper (Cu) ions, and zinc (Zn) ions. For the pet animal such as dogs and cats, it is preferable to use the former two compounds of TEA and EGTA together. To detect only the Mg ions, it is preferable to use a separate masking agent substance for the Ca ions, for example, a sulfate ion doner like Na₂ SO₄, together with any of the aforementioned masking compounds for the interfering metal ions.

In the case where the masking agent is prepared to contain the triethanolamine (TEA) and the ethylene glycolbis (2-aminoethyl ether) N·N'-tetraacetic acid (EGTA), it is made sure to eliminate the influence of the interfering metal ions in the animal urine and thereby to diagnose correctly.

The composite reagent of the present invention is formed by a mixture of the above-described color reagent substance, polyoxyethylene alkylphenyl ether, and masking agent so as to allow a veterinary observable diagnosis test, and can be produced in various product forms of a water solution, a mixed powder, or a granule or tablet shaped by means of a water-soluble binder like a dextrin. The finished product of the composite reagent is very stable in every forms and reactive in the test even if used after a long period of preservation at room temperature. Incidentally, the composite reagent in the product forms of powder and solid is provided for the test as a test solution to be dissolved with water.

The composite reagent has the aforementioned three constituents mixed with 1 part by weight of the color reagent substance, 50 to 200 (preferably 80 to 150) parts by weight of the polyoxyethylene alkylphenyl ether, and 70 to 300 (preferably 100 to 200) parts by weight of the masking agent. When TEA and EGTA are used together as the masking agent, a desirable weight ratio of TEA to EGTA is 1 to 1.5 - 4.0.

In this manner, the veterinary diagnostic test is conducted simply by treating a fixed quantity of the animal urine with a test solution of the composite reagent of the invention in water. However, to prevent a change in coloration due to a change of pH value, it is desirable to add a buffer agent beforehand to the test solution of the composite reagent. Examples of such buffer agents are water-soluble phosphate such as sodium hydrogenphosphate, and alkali compounds such as sodium hydroxide. In the case where the xylylazo violet-1 for a color reagent substance is used as one constituent, the buffer agent should be prepared so that the test solution of the composite reagent can be kept at the pH value 11-12 in the coloration range of reddish violet.

### Example

| (constituents) | (parts by weight) |
|---|---|
| xylylazo violet-1 | 0.1 |
| polyoxyethylene alkylphenyl ether (Triton X-100 manufactured by E·I·Du Pont) | 10.3 |
| TEA | 5.0 |
| EGTA | 10.6 |
| sodium hydrogenphosphate | 71.6 |
| sodium hydroxide | 5.0 |
| water | 897.4 |

A test solution of the composite reagent for a veterinary diagnostic test was prepared by a mixture of the above recited constituents and is a clear colorless liquid of pH value 11.7. The test was conducted on each urine taken out of the animals as listed hereinafter. 0.5 milliliter of each urine was treated and mixed with the test solution each separated by 10 milliliter. After the passage of five minutes, the coloration or color change of each treated solution was observed by sight, wherein the degree of the color saturation of each solution was determined by comparison with a chart representing grades of color saturation (the higher the grade of color saturation becomes, the greater the plus number becomes). The result of such observation and determination is listed in the following Table 1 together with the names of diseases or disorders diagnosed clinically by a veterinarian, the amount of magnesium (Mg) in the respective urines measured by means of titan yellow, and the presence of the urinary calculus confirmed by dropping ammonia (the sign + means existence, the sign - means absence). The aforementioned urine each was obtained out of the respective animals by compression of the urinary bladder, ureteral catheterization, or spontaneous discharge of urine.

**Table 1**

| Animals ; Age | Diseases or Disorders | Colors and Grades of saturation | Amount of Mg (milligram/one day) | urinary calculus |
|---|---|---|---|---|
| Cat A;7 | Urinary retention | reddish violet + 1.5 | 1.1 | + |
| Cat B;5 | Urinary retention | reddish violet + 1.5 | 4.2 | + |
| Cat C;3 | Urinary retention | reddish violet + 2.5 | 1.9 | + |
| Cat D;8 | Urinary retention | reddish violet + 1.8 | 0.5 | + |
| Cat E;5 | Urinary retention | reddish violet + 0.6 | 0.6 | + |
| Cat F;3 | Urinary retention | reddish violet + 1.8 | 7.0 | + |
| Cat G;3 | Urinary retention | reddish violet + 4.0 | 19.0 | + |
| Cat H;5 | Cystitis | reddish violet + 2.0 | 3.3 | + |
| Cat I;2 | Normal health (*1) | No coloration | below 0.1 | - |
| Cat J;4 | Normal health | No coloration | below 0.1 | - |
| Cat K;2 | Normal health | No coloration | below 0.1 | - |
| Dog A;5 | Urinary retention | No coloration (*2) | 2.4 | + |
| Dog B;10 | Urinary retention | reddish violet + 1.0 | 0.6 | + |

| | | | | |
|---|---|---|---|---|
| (*1) The urine was taken from the cat I neutered two months before. | | | | |
| (*2) No coloration in five minutes, but the color of reddish violet of pH11.5 was produced in ten minutes. | | | | |

The above Table I shows that the animals of normal health had their urine unchanged in color by the test solution of the composite reagent of the invention, whereas the animals, who had suffered from the disorders or diseases of the urinary tract, had their urine changed in color by the test solution while the degree of color saturation of the urine had a relationship to the amount of magnesium (Mg) in the urine. Therefore, it has become clear that the presence and degree of the aforementioned disorders of the animals can be diagnosed simply by the external observation of the treated solution and its degree of color saturation. In addition, it has become clear that the presence of a urinary calculus can be diagnosed since the urinary calculus contains a struvite (magnesium salt) and an apatite (calcium phosphate) as well as a calcium oxalate.

## Claims

1. A composite reagent, for a veterinary diagnostic test, containing a color reagent substance to produce a color by reacting with either one or both of magnesium (Mg) ions and calcium (Ca) ions, a polyoxyethylene alkylphenyl ether, and a masking agent for interfering metal ions in a urine of an animal other than the Mg ions and the Ca ions.

2. A composite reagent, for veterinary diagnostic test, as defined in claim 1, wherein the composite reagent is formed with 1 part by weight of the color reagent substance, 50 to 200 parts by weight of the polyoxyethylene alkylphenyl ether, and 70 to 300 parts by weight of the masking agent.

3. A composite reagent, for veterinary diagnostic test, as defined in claim 1, wherein the composite reagent is formed with 1 part by weight of the color reagent substance, 80 to 150 parts by weight of the polyoxyethylene alkylphenyl ether, and 100 to 200 parts by weight of the masking agent.

4. A composite reagent, for veterinary diagnostic test, as defined in anyone of claim 1 to 3, wherein a xylylazo violet-1 is used as said color reagent substance.

5. A composite reagent, for veterinary diagnostic test, as defined in any one of claims 1 to 4 wherein a polyoxyethylene isooctylphenyl ether is used as said polyoxyethylene alkylphenyl ether.

6. A composite reagent, for veterinary diagnostic test, as defined in any one of claims 1 to 4 wherein said polyoxyethylene alkylphenyl ether has n = 9 or 10 in general formula (CH₂CH₂O)ₙH of polyoxyethylene.

7. A composite reagent, for veterinary diagnostic test, as defined in any one of the previous claims wherein a triethanolamine and an ethylene glycolbis (2-aminoethyl ether) N·N'-tetraacetyl acid are used together as said masking agent.

8. A composite reagent, for veterinary diagnostic test, as defined in claim 7, wherein a weight ratio of the triethanolamine to the ethylene glycolbis (2-aminoethyl ether) N·N'-tetraacetic acid is 1 to 1.5 - 4.0.

9. A method for the diagnosis of diseases of the urinary tract in animals, characterized by the following steps:
i) Adding the reagent as defined in any one of claims 1 to 8 to a sample of animal urine, and
ii) observing the colour of the resulting mixture.

10. The use of a reagent as defined in any one of claims 1 to 8 in the diagnosis of diseases of the urinary tract in animals.
